(19) Europäisches Patentamt
European Patent Office
Office européen des brevets

(11) Veröffentlichungsnummer: **0 168 352**
**B1**

(12) # EUROPÄISCHE PATENTSCHRIFT

(45) Veröffentlichungstag der Patentschrift: **04.01.89**

(51) Int. Cl.⁴: **C 07 D 253/06**, A 01 N 43/707

(21) Anmeldenummer: **85810312.0**

(22) Anmeldetag: **08.07.85**

(54) **Verfahren zur Herstellung von Aminotriazinon-Derivaten.**

(30) Priorität: **12.07.84 CH 3403/84**

(43) Veröffentlichungstag der Anmeldung:
**15.01.86 Patentblatt 86/3**

(45) Bekanntmachung des Hinweises auf die Patenterteilung:
**04.01.89 Patentblatt 89/1**

(84) Benannte Vertragsstaaten:
**AT BE CH DE FR GB IT LI NL SE**

(56) Entgegenhaltungen:
**EP-A-0 015 452**

**CHEMICAL ABSTRACTS, vol. 100, no. 25, 18. Juni 1984, Columbus, Ohio, USA, SUMITOMO CHEMICAL CO., LTD., "6-substituted-3-dimethylamino-4-methyl-1,2-4-triazin-5-ones", p. 604, Zusammenfassung Nr. 209 879w & Jpn. Kokai Tokkyo Koho JP 58 216 173 82 216 173**
**CHEMICAL ABSTRACTS, vol. 98, no. 21, 23. Mai 1983, Columbus, Ohio, USA, EL-BAHAIE, S.A.; BADAWY, M.A.; ABDEL-HADY, S.A.; IBRAHIM, Y.A. "Rearrangement and alkylation of some 1,2,4-triazine derivatives", p. 656, Zusammenfassung Nr. 179 331g**
**CHEMICAL ABSTRACTS, Vol. 99, no. 3, 18 July 1983, Columbus , Ohio, USA SUMITOMO CHEMICAL CO: LTD. "5-Oxo-2, 5-dihydro-1,2,4-triazine derivatives as herbicides" Seite 625, Spalte 1,**

(73) Patentinhaber: **CIBA- GEIGY AG, Klybeckstrasse 141, CH- 4002 Basel (CH)**

(72) Erfinder: **Böhner, Beat, Dr., Hügelweg 3, CH- 4102 Binningen (CH)**

(56) Entgegenhaltungen: (Fortsetzung)
**Zusammenfassung Nr. 22 505q & Jpn. Kokai Tokkyo Koho JP 58 38 270 83 38 270**

**Die Akte enthält technische Angaben, die nach dem Eingang der Anmeldung eingereicht wurden und die nicht in dieser Patentschrift enthalten sind.**

**EP 0 168 352 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die nach dem erfindungsgemässen Verfahren herstellbaren Triazinon-Derivate sind teils neu, teils zusammen mit einem Verfahren zu ihrer Herstellung in der europäischen Patentpublikation EP 15 452 und dem US-Patent 3 544 570 beschrieben. Nach einem In diesen Patentpublikationen offenbarten Verfahren erfolgt die Herstellung von Aminotriazinonen durch Umsetzung eines eine substituierte Mercaptogruppe tragenden Triazinons mit einer gegebenenfalls substituierten Aminogruppe oder einem stickstoffhaltigen Heterocyclus. Ferner wird in der europäischen Patentpublikation ein Verfahren zur Herstellung von Aminotriazinon-Derivaten beschrieben, bei welchem eine α-Ketocarbonsäure mit einem 3-Amino-1,1,2-trimethyl-guanidinium-Salz, welches aus einem 1,1,2,3-Tetramethyl-isothiuronium-Salz durch Umsetzung mit Hydrazinhydrat hergestellt worden ist, umgesetzt wird.

Bei den bekannten Verfahren erfolgt also im Verlauf der Synthese die Abspaltung einer substituierten Mercaptogruppe, gewöhnlich einer Methylmercaptogruppe, woraus ökologische Probleme resultieren. Da ausserdem die Methylmercaptogruppe nur eine mässig reaktive Abgangsgruppe ist, verlaufen die Umsetzungen mit Aminen oft nur bei hohen Temperaturen. Dies bedeutet in vielen Fällen schlechte Ausbeuten bzw. unreine Reaktionsprodukte.

Es ist nun ein Ziel der vorliegenden Erfindung, ein Verfahren bereitzustellen, welches den Anfall von umweltbelastenden Nebenprodukten wermeidet und die Aminotriazinon-Derivate in guten Ausbeuten und in reiner Form liefert.

Es wurde nun gefunden, dass überraschenderweise Aminotriazinon-Derivate auf andere, ökologisch vorteilhaftere Weise herstellbar sind.

Erfindungsgemäss wird somit vorgeschlagen, Aminotriazinon-Derivate der Formel I

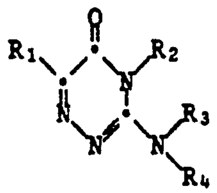

(I),

worin

$R_1$ und $R_2$ unabhängig voneinander Alkyl, welches unsubstituiert oder durch Alkoxy oder Halogen substituiert ist, Cycloalkyl, welches unsubstituiert oder durch Methyl substituiert ist, Aralkyl, welches unsubstituiert oder durch Substituenten aus der Gruppe Halogen, Alkyl, Halogenalkyl und Alkoxy ein- oder mehrfach substituiert ist, oder Aryl, welches unsubstituiert oder durch Substituenten aus der Gruppe Alkyl, Halogen, Halogenalkyl und Alkoxy ein- oder mehrfach substituiert ist, und

$R_3$ und $R_4$ unabhängig voneinander Wasserstoff, Alkyl oder unsubstituiertes oder durch Methyl substituiertes Cycloalkyl oder gemeinsam mit dem Stickstoffatom, an welches sie gebunden sind, einen Piperidin- oder Morpholinring bedeuten, in der Weise herzustellen, dass man eine Verbindung der Formel II

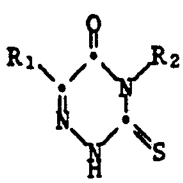

(II),

worin $R_1$ und $R_2$ die vorstehend angegebenen Bedeutungen haben, mit Phosgen umsetzt und die so erhaltene Verbindung der Formel III

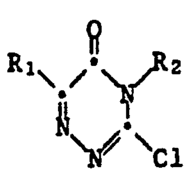

• HCl (III),

worin $R_1$ und $R_2$ die vorstehend angegebenen Bedeutungen haben, ohne Isolierung oder nach Isolierung mit einer Verbindung der Formel IV

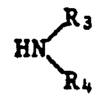

(IV),

worin $R_1$ und $R_4$ die vorstehend angegebenen Bedeutungen haben, umsetzt.

In den Verbindungen der Formel I als Substituenten $R_1$, $R_2$, $R_3$ oder $R_4$ oder als Teil der Substituenten $R_1$ und $R_2$ vorliegende Alkylgruppen können geradkettig oder verzweigt sein und enthalten im allgemeinen 1 bis 8, vorzugsweise 1 bis 5 Kohlenstoffatome. Hierzu gehören Methyl, Äthyl, n-Propyl, Isopropyl, n-Butyl, Isobutyl, sek.-Butyl und tert.-Butyl sowie n-Pentyl, n-Hexyl, n-Heptyl, n-Oktyl und ihre Isomere.

Als Alkoxygruppen kommen vorzugsweise solche mit 1 bis 4 Kohlenstoffatomen in Betracht, also Methoxy, Äthoxy, n-Propoxy, Isopropoxy, n-Butoxy, Isobutoxy, sek.-Butoxy und tert.-Butoxy.

Halogen als Substituent oder Teil eines Substituenten steht für Fluor, Chlor, Brom und Jod, insbesondere Fluor und Chlor.

Halogenalkylgruppen enthalten in der Regel 1 bis 4, vorzugsweise 1 oder 2 Kohlenstoffatome und können einfach bis vollständig halogeniert sein. Vorzugsweise enthalten sie 1 bis 3 Halogenatome. Beispiele für Halogenalkylgruppen sind 1,1-Dimethyl-2-chloräthyl, 1-Methyl-3-fluorpropyl, Trichlormethyl, 3,3,3-Trifluorpropyl, 2,3-Dichlor-2-methylpropyl, 1-(Chlormethyl)-äthyl, Trifluormethyl, 1-(Fluormethyl)-äthyl- 1,1-Dimethyl-3-chlorpropyl, 1-Methyl-3-chlorpropyl und 1,1-Dimethyl-2-fluoräthyl.

Arylgruppen enthalten im allgemeinen 6 bis 18 Kohlenstoffatome Bevorzugt sind Phenyl und Naphthyl.

Als Aralkylgruppen kommen vor allem solche mit 6 bis 10 Kohlenstoffatomen im aromatischen Teil und 1 bis 4 Kohlenstoffatomen im Alkylteil in Frage, insbesondere Benzyl.

Aryl als Substituent oder Teil eines Substituenten kann durch Substituenten der Gruppe Halogen, Alkyl, Halogenalkyl und Alkoxy substituiert sein, und zwar vorzugsweise durch 1 bis 3 der genannten Substituenten.

Beispiele für substituierte Arylgruppen sind 2-Methoxy-4-chlorphenyl, 3-Chlor-4-trifluormethylphenyl, 2,6-Dimethoxyphenyl, 2,4,6-Trifluorphenyl, 2-Chlor-4-äthoxyphenyl, 2,4-Dimethoxyphenyl, 2-Chlor-4-trifluormethylphenyl, 2,4-Difluorphenyl, 4-Chlormethylphenyl, 3-Dichlormethylphenyl, 2-Chlor-4-methoxyphenyl, 4-Methoxy-α-naphthyl, 6,7-Dichloro-α-naphthyl und 3-Chlormethyl-β-naphthyl; Beispiele für substituierte Aralkylgruppen sind 2-(2-Chlor-4-triflourmethylphenyl)-äthylt 4-Methoxybenzyl, 2,4,6-Trifluorbenzyl, 2-(2,4-Dimethoxyphenyl)-äthyl, 2-Chlor-4-trifluormehylbenzyl, 3,5-Dichlorbenzyl und 4-Trifluormethylbenzyl.

Cycloalkyl steht für Cyclopropyl, Cyclobutyl, Cyclopentyl, Cyclohexyl, Cycloheptyl und Cyclooktyl. Die Cycloalkylgruppen sind unsubstituiert oder durch Methyl substituiert. Bevorzugt enthalten die Cycloalkylgruppen 1 bis 2 Methylgruppen.

Die Umsetzung einer Verbindung der Formel II, welche auch in tautomerer Form als

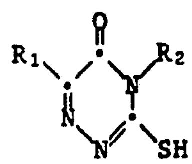

vorliegen kann, mit Phosgen erfolgt zweckmässigerweise in einem gegenüber Phosgen inerten Lösungs- oder Verdünnungsmittel bei einer Temperatur im Bereich von 0 bis 80°C, insbesondere 20 bis 50°C, und bei Normaldruck.

Im allgemeinen lässt sich eine vollständige Umsetzung erzielen, wenn das Reaktionsgemisch während 14 bis 16 Stunden bei etwa 20 bis 25°C oder während 1 bis 2 Stunden bei etwa 45°C ausgerührt wird.

Als Lösungsmittel eignen sich insbesondere aromatische und aliphatische Kohlenwasserstoffe, wie beispielsweise Benzol und Toluol, halogenierte Kohlenwasserstoffe, wie beispielsweise Chloroform und Tetrachlorkohlenstoff, sowie Ester organischer Säuren wie beispielsweise Äthylacetat.

Es erweist sich als besonders vorteilhaft, ein Lösungsmittel zu verwenden, in welchem die Hydrochloride der 3-Chlortriazinone der Formel III schwer löslich sind, da diese so durch Filtration leicht isolierbar sind.

Bei der Umsetzung von Verbindungen der Formel II mit Phosgen fallen die entstehenden 3-Chlortriazinone in Form von Hydrochloriden an und können als solche umgesetzt werden. Die freie Form der 3-Chlortriazinone lässt sich sehr leicht durch Sublimieren, beispielsweise im Hochwakuum, erzeugen.

Die Umsetzung einer Verbindung der Formel III in Form ihres Hydrochlorids oder als freie Verbindung mit einer Verbindung der Formel IV erfolgt zweckmässigerweise in einem gegenüber den Reaktionsteilnehmern inerten Lösungs- oder Verdünnungsmittel und in Gegenwart einer Base, sowie bei einer Temperatur im Bereich von 0 bis 150°C, insbesondere 20 bis 80°C.

Als Lösungsmittel besonders geeignet sind aliphatische und aromatische Kohlenwasserstoffe, wie beispielsweise Benzol, Toluol, Xylole. Petroläther, Cyclohexan, n-Hexan; halogenierte Kohlenwasserstoffe wie beispielsweise Chlorbenzol, Methylenchlorid, Äthylenchlorid, Chloroform, Tetrachlorkohlenstoff, Tetrachloäthylen; Äther und ätherartige Verbindungen wie beispielsweise Dialkyläther, wie Diäthyläther und

Diisopropyläther; oder Gemische solcher Lösungsmittel untereinander.

Geeignete Basen sind im allgemeinen anorganische Basen aus der Reihe: Hydride wie beispielsweise Natriumhydrid oder Calciumhydrid; Oxide, wie beispielsweise Magnesiumoxid, Calciumoxid oder Natriumoxid; Hydroxide wie beispielsweise Lithiumhydroxid, Natriumhydroxid oder Kaliumhydroxid; oder Carbonate wie beispielsweise Natriumcarbonat oder Kaliumcarbonat; oder organische Basen wie beispielsweise Triäthylamin, Trimethylamin, Pyridin oder das Amin der Formel IV im Überschuss.

Die Ausgangssubstanzen der Formeln II und IV sind bekannt oder lassen sich analog bekannten Verfahren herstellen.

Die Aminotriazinon-Derivate der Formel I sind zum Teil ohne und zum Teil bereits bekannte Verbindungen.

Die Verbindungen der Formel I weisen eine ausgeprägte herbizide Aktivität auf. Wegen ihrer herausragenden Wirkung sind insbesondere 6-Cyclohexyl-4-methyl-3-methylamino-1,2,4-triazin-5(4H)-on, 6-tert.-Butyl-4-methyl-3-methylamino-1,2,4-triazin-5(4H)-on und ganz besonders 3-Dimethylamino-6-isopropyl-4-methyl-1,2,4-triazin-5(4H)-on zu erwähnen.

Zur Applikation als Herbizide werden die Verbindungen der Formel I in unveränderter Form oder vorzugsweise zusammen mit den in der Formulierungstechnik üblichen Hilfsmitteln eingesetzt und werden daher z. B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, auch Verkapselungen in z. B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt.

Die formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z. B. Xyolgemische oder substituierte Naphthaline, Phthalsäureester wie Dibutyl- oder Dioctylphthalat, aliphatische Kohlenwasserstoffe wie Cyclohexan oder Paraffine. Alkohole und Glykole sowie deren Äther und Ester, wie Äthanol, Äthylenglykol, Äthylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z. B. Bimsstein, Ziegelbruch, Sepiolit oder Bentonit, als nicht sorptive Trägermaterialien z. B. Calcit oder Sand in Frage. Darüberhinaus können eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z. B. die Na- oder K-Salze der Öl- oder Stearinsäure, oder von natürlichen Fettsäuregemischen genannt, die z. B. aus Kokosnuss- oder Talgöl gewonnen werden können. Ferner sind auch die Fettsäuremethyllaurinsalze zu erwähnen.

Häufiger werden jedoch sog. synthetische Tenside verwendet, insbesondere Fettsulfonate, Fettsulfate, sulfonierte Benzimidazolderivate oder Alkylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z. B. aus Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Äthylenoxid-Addukten. Die sulfonierten Benzimidazolderivate enthalten vorzugsweise 2 Sulfonsäuregruppen und einen Fettsäurerest mit 8-22 C-Atomen. Alkylarylsulfonate sind z. B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z. B. Salze des Phosphorsäureesters eines p-Nonylphenol-(4-14)-Äthylenoxid-Adduktes oder Phospholipide in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Clykolläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Äthylenglykoläthergruppen und 10 bis 100 Propylenglykoläthergruppen enthaltenden Polyäthylenoxidaddukte an Polypropylenglykol,

Äthylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Äthylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Äthylsulfate vor, z. B. das Stearyltrimethylammoniumchlorid und das Benzyldi-(2-chlorätyl)äthylammoniumbromid.

Die in der Formulierungstechnik gebräuchlichen Tenside sind u.a. in folgenden Publikationen beschrieben:

"Mc Cutcheon's Detergents and Emulsifiers Annual" MC
Publishing Corp., Ridgewood New Jersey, 1980
Stache, H., "Tensid-Taschenbuch",
Carl Hanser Verlag, München/Wien, 1981

Die agrochemischen Zubereitungen enthalten in der Regel 0,1 bis 99 %, insbesondere 0,1 bis 95 %, Wirkstoff der Formel 1, 99,9 bis 1 %, insbesondere 99,8 bis 5 %, eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Aminotriazinon-Derivate der Formel Ia, insbesondere 3-Dimethylamino-6-isopropyl-4-methyl-1,2,4-triazin-5(4H)-on, sind zur Bekämpfung monokotyler und dikotyler Unkräuter ausgezeichnet geeignet, und zwar sowohl bei der Anwendung im Vorauflaufverfahren (pre-emergent) wie im Nachauflaufverfahren (post-emergent).

Besonders vorteilhaft lassen sich die Aminotriazinon-Derivate der Formel 1a oder sie enthaltende Mittel zur selektiven Bekämpfung von Unkräutern in Nutzpflanzenkulturen einsetzen, wie beispielsweise in Kulturen von Getreide, Soja, Kartoffeln, Tomaten und Zuckerrohr.

Die Aufwandmengen, in denen die Substanzen anzuwenden sind, hängen von den jeweiligen Gegebenheiten, wie insbesondere den Witterungsbedingungen, der Bodenbeschaffenheit, dem Pflanzenbewuchs und dem Zeitpunkt der Applikation ab. Als zweckmässig erweisen sich im allgemeinen Aufwandmengen an Wirksubstanz von 30 bis 2 000 g/Hektar, insbesondere 100 bis 500 g/Hektar.

**Beispiel 1**

Herstellung von 3-Chlor-6-isopropyl-4-methyl-1,2,4-triazin-5(4H)-on-hydrochlorid

Zu einer Lösung von 34,6 g (0,35 M) Phosgen in 600 ml Essigsäure werden 60 g (0,35 M) 6-Isopropyl-4-methyl-3-mercapto-1,2,4-triazin-5(4H)-on gegeben. Nach dem Abklingen der leicht exothermen Reaktion wird noch eine Stunde bei 25°C ausgerührt. Der entstandene Niederschlag wird abfiltriert und mit wenig Essigsäureäthylester gewaschen. Man erhält 64.5 g (90 % d.Th.) 3-Chlor-6-isopropyl-4-methyl-1,2,4-triazin-5(4H)-on-hydrochlorid. Smp. 120°C (Z).

Durch anschliessende Sublimierung des erhaltenen Produkts im Hochvakuum lässt sich das freie 3-Chlor-6-isopropyl-4-methyl-1,2,4-triazin-5(4H)-on herstellen. Smp. 144°C (Z).

**Beispiel 2**

Herstellung von 3-Chlor-6-isopropyl-4-methyl-1,2,4-triazin-5(4H)-on-hydrochlorid

18,6 g (0,1 M) 6-Isopropyl-3-mercapto-4-methyl-1,2,4-triazin-5(4H)-on werden in 100 ml Essigester suspendiert. Bei Raumtemperatur leitet man nun 10,9 g (0,11 M) Phosgen ein, wobei sich das Reaktionsgemisch leicht erwärmt. Da die Umsetzung noch nicht vollständig ist, lässt man noch 1 Stunde bei 45°C ausrühren. Das Gemisch, welches nach wie vor als weisse Suspension vorliegt, wird nun filtriert. Den Rückstand wäscht man mit absolutem Diäthyläther und trocknet das weisse, pulvrige Produkt im Vakuum ohne Wärmezufuhr. So erhält man 20,3 g (90,8 % d.Th.) 3-Chlor-6-isopropyl-4-methyl-1,2,4-triazin-5(4H)-on-hydrochlorid mit einem Smp. von 144°C (Z). Beim Erwärmen auf 45°C am Hochvakuum sublimiert die Verbindung und man erhält das freie Chlorid mit einem Smp. von 129°C (Z).

Auf analoge Weise lassen sich auch die folgenden, in der Tabelle 1 zusammen mit der Verbindung des Beispiels 1 aufgeführten Verbindungen der Formel III herstellen.

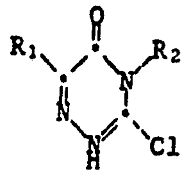

• HCl

**Tabelle 1**

| Verbindung Nr. | $R_1$ | $R_2$ | physikalische Daten |
|---|---|---|---|
| 1.1 | iso$C_3H_7$ | $CH_3$ | Smp- 144° C (Z) |
| 1.2 | 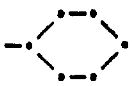 | $CH_3$ | Smp. 154-155° C |
| 1.3 | 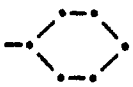 | $C_2H_5$ | Smp. 134-135° C |
| 1.4 | tert.-$C_4H_9$ | $CH_3$ | Smp. 160° C (Z) |
| 1.5 | 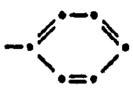 | $CH_3$ | Smp. 118° C (Z) |
| 1.6 | sek.-$C_4H_9$ | $CH_3$ | Smp. 121° C (Z) |
| 1.7 | $CH(C_2H_5)_2$ | $CH_3$ | Smp. 112° C (Z) |
| 1.8 | $CH_3$ | $CH_3$ | Smp. 165° C (Z) |
| 1.9 | tert.$C_4H_9$ | $C_2H_9$ | Smp. 148° C (Z) |
| 1.10 | $C_2H_5$ | $CH_3$ | |
| 1.11 | $C_2H_5$ | $C_2H_5$ | |
| 1.12 | n-$C_3H_5$ | $CH_3$ | |
| 1.13 | iso-$C_3H_7$ | $C_2H_5$ | |
| 1.14 | $CH_3$ | $C_2H_5$ | |
| 1.15 | $CH_3$ | n-$C_3H_7$ | |
| 1.16 | $CH_3$ | iso-$C_3H_7$ | |
| 1.17 | $CH_3$ | n-$C_4H_9$ | |
| 1.18 | 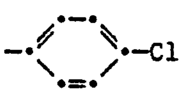 | $CH_3$ | |
| 1.19 | 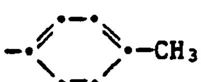 | $CH_3$ | |
| 1.20 | 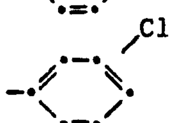 | $CH_3$ | |
| 1.21 | 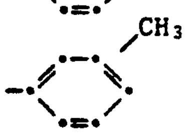 | $CH_3$ | |
| 1.22 | 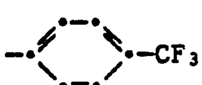 | $CH_3$ | |
| 1.23 | 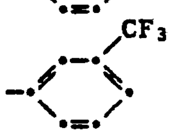 | $CH_3$ | |

| | | |
|---|---|---|
| 1.24 | 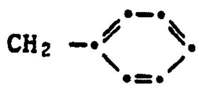 | $CH_3$ |
| 1.25 | 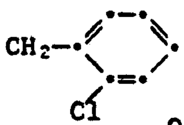 | $CH_3$ |
| 1.26 | 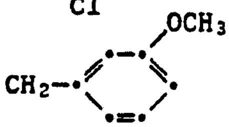 | $CH_3$ |
| 1.27 | 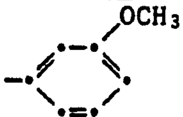 | $CH_3$ |
| 1.28 | 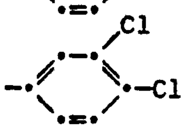 | $CH_3$ |

**Beispiel 3**

Herstellung von 3-Dimethylamino-6-isopropyl-4-methyl-1,2,4-triazin-5(4H)-on

30,0 g (0,15 M) 3-Chlor-6-isopropyl-4-methyl-1,2,4-triazin-5(4H)-on-hydrochlorid werden in 60 ml Toluol suspendiert. Zu diesem gerade noch rührbaren Gemisch lässt man unter leichter Kühlung bei ca. 30°C 20,4 g (0,45 M) Dimethylamin gelöst in 80 ml Toluol innert 2 Min. zulaufen. Dann entfernt man die Kühlung, worauf sich das Reaktionsgemisch auf 50°C erwärmt. Nach 15 Min. ausrühren ist die Reaktion beendet. Das Gemisch wird mit 50 ml kaltem Wasser gut durchgerührt, die organische Phase abgetrennt, mit Magnesiumsulfat getrocknet und zur Trockene eingedampft. Dabei erhält man ein hellbeiges, klares Öl in quant. Ausbeute. Nach der Destillation fallen 21,4 g (72,8 %) 3-Dimethylamino-6-isopropyl-4-methyl-1,2,4-triazin-5(4H)-on als farbloses, klares Öl mit einem Sp. 135°C/0.04 mm an.

Auf analoge Weise lassen sich auch die folgenden, in den Tabellen 2 und 3 zusammen mit der Verbindung des vorstehenden Beispiels aufgeführten Verbindungen der Formel I herstellen.

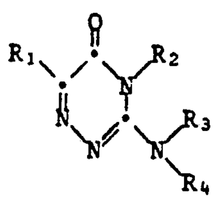

**Tabelle 2** (neue Verbindungen)

| Verbindung Nr. | $R_1$ | $R_2$ | $R_3$ | $R_4$ | physikal. Daten |
|---|---|---|---|---|---|
| 2.1 | Cyclohexyl | $CH_3$ | H | $CH_3$ | Smp. 160-161°C |
| 2.2 | tert.-$C_4C_9$ | $CH_3$ | H | $CH_3$ | Smp. 197-198°C |
| 2.3 | iso$C_3H_7$ | $CH_3$ | $CH_3$ | $CH_3$ | $n^{20}_D = 1.5362$ |
| 2.4 | Cyclohexyl | $CH_3$ | H | $C_2H_5$ | Smp. 165-167°C |
| 2.5 | $CH(C_2H_5)_2$ | $CH_3$ | H | $CH_3$ | Smp. 125-126°C |
| 2.6 | sek.-$C_4H_9$ | $CH_3$ | H | $CH_3$ | Smp. 129-130°C |

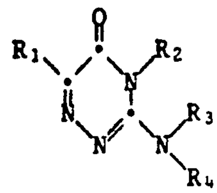

**Tabelle 3** (bekannte Verbindungen)

| Verbindung Nr. | $R_1$ | $R_2$ | $R_3$ | $R_4$ | physikal. Daten |
|---|---|---|---|---|---|
| 3.1 | iso$C_3H_7$ | $CH_3$ | H | $CH_3$ | Smp. 109-110° C |
| 3.2 | $C_2H_5$ | $CH_3$ | Piperidino | | Smp. 132° C |
| 3.3 | tert.-$C_4H_9$ | $CH_3$ | $CH_3$ | $CH_3$ | Smp. 87-88° C |
| 3.4 | Cyclohexyl | $CH_3$ | $CH_3$ | $CH_3$ | Smp. 104-106° C |
| 3.5 | sek.-$C_4H_9$ | $CH_3$ | $CH_3$ | $CH_3$ | $n^{20}_D = 1,5325$ |
| 3.6 | $CH(C_2H_5)_2$ | $CH_3$ | $CH_3$ | $CH_3$ | |
| 3.7 | 1-Methylcyclobutyl | $CH_3$ | $CH_3$ | $CH_3$ | |
| 3.8 | Cycloheptyl | $CH_3$ | $CH_3$ | $CH_3$ | |
| 3.9 | Phenyl | $CH_3$ | H | $CH_3$ | Smp. 227-229° C |
| 3.10 | Phenyl | $CH_3$ | H | H | Smp. 270° C |
| 3.11 | Phenyl | $CH_3$ | Morpholino | | Smp. 130° C |
| 3.12 | Phenyl | $CH_3$ | n-$C_4H_9$ | n-$C_4H_9$ | Smp. 135-145° C |
| 3.13 | Phenyl | $CH_3$ | $CH_3$ | $CH_3$ | Smp. 111-112° C |

**Formulierungsbeispiele für flüssige Wirkstoffe der Formel I** (% = Gewichtsprozent)

| 4. Emulsions-Konzentrat | a) | b) | c) |
|---|---|---|---|
| Wirkstoff aus Tabelle 2 | 25 % | 40 % | 50 % |
| Ca-Dodecylbenzolsulfonat | 5 % | 8 % | 6 % |
| Ricinusölpolyäthylglykoläther (36 Mol ÄO) | 5 % | - | - |
| Tributylphenolpolyäthenglykoläther (30 Mol ÄO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 5. Lösungen | a) | b) | c) | d) |
|---|---|---|---|---|
| Wirkstoff aus Tabelle 2 | 80 % | 10 % | 5 % | 95 % |
| Äthylenglykolmonomethyläther | 20 % | - | - | - |
| Polyäthylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenze 160-190° C) | - | - | 94 % | - |

Die Lösungen sind zur Anwendung in Form kleinster Tropfen geeignet.

| 6. Granulate | a) | b) |
|---|---|---|
| Wirkstoff aus Tabelle 2 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 7. Stäubemittel | a) | b) |
|---|---|---|
| Wirkstoff aus Tabelle 2 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

**Formulierungsbeispiele für feste Wirkstoffe der Formel I** (% = Gewichtsprozent)

| 8. Spritzpulver | a) | b) | c) |
|---|---|---|---|
| Wirkstoff aus Tabelle 2 | 25 % | 50 % | 75 % |
| Na-Ligninsulfonat | 5 % | 5 % | - |
| Na-Laurylsulfonat | 3 % | - | 5 % |
| Na-Diisopropylnaphthalinsulfonat | - | 6 % | 10 % |
| Octylphenolpolyäthylenglykoläther (7-8 Mol ÄO) | | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 9. Emulsions-Konzentrat | |
|---|---|
| Wirkstoff aus Tabelle 2 | 10 % |
| Octylphenolpolyäthylenglykoläther (4-5 Mol ÄO) | 3 % |
| Ca-Dodecylbenzolsulfonat | 3 % |
| Ricinusölpolyglykoläther (35 Mol ÄO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 10. Stäubemittel | a) | b) |
|---|---|---|
| Wirkstoff aus Tabelle 2 | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

| 11. Extruder-Granulate | |
|---|---|
| Wirkstoff aus Tabelle 2 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| 12. Umhüllungs-Granulat | |
|---|---|
| Wirkstoff aus Tabelle 2 | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

13. Suspensions-Konzentrat

| | |
|---|---|
| Wirkstoff aus Tabelle 2 | 40 % |
| Äthylenglykol | 10 % |
| Nonylphenolpolyäthylenglykoläther (15 Mol ÄO) | 6 % |
| Na-Ligninsulfonat | 10 % |
| Carboxymethylcellulose | 1 % |
| 37 %-ige wässrige Formaldehyd-Lösung | 0,2 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

**Biologische Beispiele**

**Beispiel 14:** Pre-emergente Herbizid-Wirkung

Im Gewächshaus wird unmittelbar nach der Einsaat der Versuchspflanzen in Töpfe von 11 cm Durchmesser die Erdoberfläche mit einer wässrigen Wirkstoffemulsion in einer Dosierung, welche 4 kg Wirksubstanz pro Hektar entspricht, behandelt. Die angesäten Töpfe werden im Gewächshaus bei 20 bis 24°C und 50 bis 70 % relativer Luftfeuchtigkeit gehalten. Der Versuch wird nach 3 Wochen ausgewertet und die Resultate werden nach folgender Notenskala bonitiert:

1 = Pflanze nicht gekeimt oder total abgestorben
2-3 = sehr starke Wirkung
4-6 = mittlere Wirkung
7-8 = geringe Wirkung
9 = keine Wirkung (wie unbehandelte Kontrolle)

Die Resultate zeigt die nachfolgende Tabelle:

**Tabelle 4:** Pre-emergente Wirkung

| Verb. Nr. | Avena | Setaria | Sinapis | Stellaria |
|---|---|---|---|---|
| 2.1 | 3 | 2 | 1 | 2 |
| 2.2 | 1 | 1 | 1 | 1 |
| 2.3 | 1 | 1 | 1 | 1 |
| 2.4 | 1 | 1 | 1 | 1 |
| 2.5 | 1 | 1 | 1 | 1 |
| 2.6 | 1 | 1 | 1 | 1 |

**Beispiel 15:** Post-emergente Herbizid-Wirkung

Im Gewächshaus werden in Töpfen von 11 cm Durchmesser die Pflanzen Soja, Avena fatua, Setaria, Lolium, Solanum, Stellaria, Sinapis und Phaseolus gezogen, bis sie das 3-6 Blatt-Stadium erreicht haben, was nach ca. 2 Wochen der Fall ist. Dann werden sie mit einer wässrigen Wirkstoffemulsion in einer Dosierung, welche 4 kg Wirksubstanz pro Hektar entspricht, gespritzt und dann bei 20-24°C und 45-60 % relativer Luftfeuchtigkeit gehalten. 15 Tage nach der Behandlung wird der Versuch ausgewertet und das Ergebnis wie im Vorauflauf-Versuch nach derselben Notenskala ausgewertet. Die Resultate sind wie folgt:

**Tabelle 5:** Post-emergente Wirkung

| Verb. Nr. | Avena | Setaria | Lolium | Solanum | Sinapis | Stellaria | Phaseolus |
|---|---|---|---|---|---|---|---|
| 2.1 | 1 | 1 | 2 | 1 | 1 | 1 | 3 |
| 2.2 | 1 | 1 | 1 | 1 | 1 | 1 | 3 |
| 2.3 | 1 | 1 | 1 | 1 | 1 | 1 | 2 |
| 2.4 | 1 | 2 | 1 | 1 | 1 | 2 | 4 |
| 2.5 | 1 | 1 | 1 | 1 | 1 | 1 | 2 |
| 2.6 | 1 | 1 | 1 | 1 | 1 | 1 | 2 |

## Patentansprüche

1. Verfahren zur Herstellung von Aminotriazinon-Derivaten der Formel I

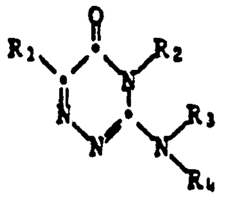

(I),

worin
$R_1$ und $R_2$ unabhängig voneinander Alkyl, welches unsubstituiert oder durch Alkoxy oder Halogen substituiert ist, Cycloalkyl, welches unsubstituiert oder durch Methyl substituiert ist, Aralkyl, welches unsubstituiert oder durch Substituenten aus der Gruppe Halogen, Alkyl, Halogenalkyl und Alkoxy ein- oder mehrfach substituiert ist, oder Aryl, welches unsubstituiert oder durch Substituenten aus der Gruppe Alkyl, Halogen, Halogenalkyl und Alkoxy ein- oder mehrfach substituiert ist, und
$R_3$ und $R_4$ unabhängig voneinander Wasserstoff, Alkyl oder unsubstituiertes oder durch Methyl substituiertes Cycloalkyl oder gemeinsam mit dem Stickstoffatom, an welches sie gebunden sind, einen Piperidin- oder Morpholinring bedeuten, dadurch gekennzeichnet, dass man eine Verbindung der Formel II

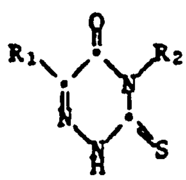

(II),

worin $R_1$, und $R_2$ die vorstehend angegebenen Bedeutungen haben, mit Phosgen umsetzt und die so erhaltene Verbindung der Formel III

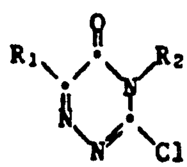

(III),

worin $R_1$, und $R_2$ die vorstehend angegebenen Bedeutungen haben, einschliesslich ihrer Hydrochloride, mit einer Verbindung der Formel IV

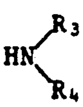

(IV),

worin $R_3$ und $R_4$ die vorstehend angegebenen Bedeutungen haben, umsetzt.

2. Verfahren nach Anspruch 1, dadurch gekennzeichnet, dass man die Umsetzung einer Verbindung der Formel II mit Phosgen in einem gegenüber Phosgen inerten Lösungs- oder Verdünnungsmittel vornimmt.

3. Verfahren nach Anspruch 1, dadurch gekennzeichnet, dass man die Umsetzung einer Verbindung der Formel II mit Phosgen bei einer Temperatur im Bereich von 0 bis 80°C vornimmt.

4. Verfahren nach Anspruch 3, dadurch gekennzeichnet, dass man die Umsetzung bei einer Temperatur im Bereich von 20 bis 50°C vornimmt.

5. Verfahren nach Anspruch 1, dadurch gekennzeichnet, dass man die Umsetzung einer Verbindung der Formel II mit Phosgen unter Normaldruck vornimmt.

6. Verfahren nach Anspruch 1, dadurch gekennzeichnet, dass man die Umsetzung einer Verbindung der Formel III mit einer Verbindung der Formel IV in einem gegenüber den Reaktionsteilnehmern inerten Lösungs- oder Verdünnungsmittel durchführt.

7. Verfahren nach Anspruch 1, dadurch gekennzeichnet, dass man die Umsetzung einer Verbindung der Formel III mit einer Verbindung der Formel IV in Gegenwart einer Base durchführt.

8. Verfahren nach Anspruch 1, dadurch gekennzeichnet, dass man die Umsetzung einer Verbindung der Formel III mit einer Verbindung der Formel IV bei einer Temperatur im Bereich von 0 bis 150° C durchführt.

9. Verfahren nach Anspruch 8, dadurch gekennzeichnet, dass man die Umsetzung bei einer Temperatur im Bereich von 20 bis 80° C durchführt.

**Claims**

1. A process for the preparation of aminotriazinone derivatives of formula I

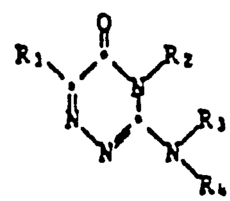

(I),

wherein

$R_1$ and $R_2$, each independently of the other, are alkyl which is unsubstituted or substituted by alkoxy or halogen; or cycloalxyl which is unsubstituted or substituted by methyl; aralkyl which is unsubstituted or substituted by one or more substituents from the group consisting of halogen, alkyl, haloalkyl and alkoxy; or are aryl which is unsubstituted or substituted by one or more substituents from the group consisting of alkyl, halogen, haloalkyl and alkoxy; and

$R_3$ and $R_4$, each independently of the other, are hydrogen, alkyl, cycloalkyl which is unsubstituted or substituted by methyl; or $R_3$ and $R_4$, together with the nitrogen atom to which they are bonded, are a piperidine or morpholine ring,

which process comprises reacting a compound of formula II

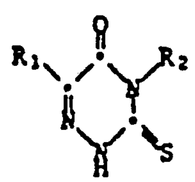

(II),

wherein $R_1$ and $R_2$ are as defined above, with phosgene, and reacting the compound of formula III so obtained

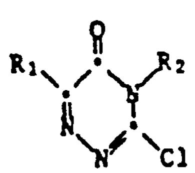

(III),

wherein $R_1$ and $R_2$ are as defined above, including the hydrochlorides thereof, with a compound of formula IV

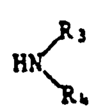

(IV),

wherein $R_3$ and $R_4$ are as defined above.

2. A process according to claim 1, wherein the reaction of a compound of formula II with phosgene is carried out in a solvent or diluent which is inert to phosgene.

3. A process according to claim 1, wherein the reaction of a compound of formula II with phosgene is carried out in the temperature range from 0° to 80° C.

4. A process according to claim 3, wherein the reaction is carried out in the temperature range from 20° to 50° C.

5. A process according to claim 1, wherein the reaction of a compound of formula II with phosgene is carried out under normal pressure.

6. A process according to claim 1, wherein the reaction of a compound of formula III with a compound of formula IV is carried out in a solvent or diluent which is inert to the reactants.

7. A process according to claim 1, wherein the reaction of a compound of formula III with a compound of formula IV is carried out in the presence of a base.

8. A process according to claim 1, wherein the reaction of a compound of formula III with a compound of formula IV is carried out in the temperature range from 0° to 150°C.

9. A process according to claim 8, wherein the reaction is carried out in the temperature range from 20° to 80°C.

**Revendications**

1. Procédé de préparation de dérivés de l'aminotriazinone répondant à la formule I

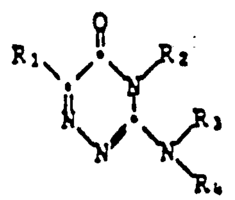

(I),

dans laquelle

$R_1$ et $R_2$ représentent chacun, indépendamment l'un de l'autre, un groupe alkyle non substitué ou substitué par un groupe alcoxy ou un halogène, un groupe cycloalkyle non substitué ou substitué par un groupe méthyle, un groupe aralkyle non substitué ou portant un ou plusieurs substituants choisis parmi les halogènes, les groupes alkyle, halogénoalkyle et alcoxy, ou un groupe aryle non substitué ou portant un ou plusieurs substituants choisis parmi les groupes alkyle, les halogènes, les groupes halogénoalkyle et alcoxy, et $R_3$ et $R_4$ représentent chacun, indépendamment l'un de l'autre, l'hydrogène, un groupe alkyle ou un groupe cycloalkyle non substitué ou substitué par un groupe méthyle, ou forment ensemble et avec l'atome d'azote auquel ils sont reliés, un cycle pipéridine ou morpholine, caractérisé en ce que l'on fait réagir un composé de formule II

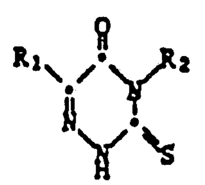

(II),

dans laquelle $R_1$ et $R_2$ ont les significations indiquées ci-dessus, avec le phosgène, ce qui donne un composé de formule III

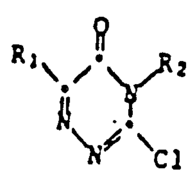

(III)

dans laquelle $R_1$ e t $R_2$ ont les significations indiquées ci-dessus, éventuellement à l'état de chlorhydrate, qu'on fait réagir avec un composé de formule IV

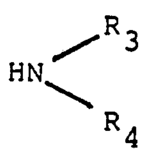

(IV),

dans laquelle $R_3$ et $R_4$ ont les significations indiquées ci-dessus.

2. Procédé selon la revendication 1, caractérisé en ce que la réaction entre un composé de formule II et le phosgène est effectuée dans un solvant ou diluant inerte à l'égard du phosgène.

3. Procédé selon la revendication 1, caractérisé en ce que la réaction entre un composé de formule II et le phosgène est effectuée à une température dans l'intervalle de 0 à 80°C.

4. Procédé selon la revendication 3, caractérisé en ce que la réaction est effectuée à une température dans l'intervalle de 20 à 50°C.

5. Procédé selon la revendication 1, caractérisé en ce que la réaction entre un composé de formule II et le phosgène est effectuée à pression normale.

6. Procédé selon la revendication 1, caractérisé en ce que la réaction entre un composé de formule III et un composé de formule IV est effectuée dans un solvant ou diluant inerte à l'égard des réactifs.

7. Procédé selon la revendication 1, caractérisé en ce que la réaction entre un composé de formule III et un composé de formule IV est effectuée en presence d'une base.

8. Procédé selon la revendication 1, caractérisé en ce que la réaction entre un composé de formule III et un composé de formule IV est effectuée à une température dans l'intervalle de 0 à 150° C.

9. Procédé selon la revendication 8, caractérisé en ce que la réaction est effectuée à une température dans l'intervalle de 20 à 80° C.